# FASCICULE DE BREVET EUROPEEN

(11) **EP 2 597 944 B1**
(45) Date de publication et mention de la délivrance du brevet: **18.01.2017**
(21) Numéro de dépôt: 11726848.2
(22) Date de dépôt: 23.06.2011
(51) Int. Cl.: A01K 11/00, A01K 13/00, A61B 10/02

(54) **ENSEMBLE DE MARQUAGE ET/OU DE PRELEVEMENT DE TISSU D'UN ANIMAL ET OUTIL DE MARQUAGE ET/OU DE PRELEVEMENT CORRESPONDANT**
EINHEIT ZUR MARKIERUNG UND/ODER PROBENAHME VON TIERGEWEWBE SOWIE ZUGEHÖRIGES MARKERIERUNGS- UND/ODER PROBENAHMEINSTRUMENT
UNIT FOR MARKING AND/OR SAMPLING ANIMAL TISSUE, AND CORRESPONDING MARKING AND/OR SAMPLING TOOL

(30) Priorité: 30.07.2010 FR 1056349
(43) Date de publication de la demande: 05.06.2013
(73) Titulaire: Allflex Europe, 35500 Vitre (FR)
(72) Inventeur: TEYCHENE, Bruno, F-81430 Mouzieys-Teulet (FR); HILPERT, Jean-Jacques, F-35500 Vitre (FR)
(74) Mandataire: Vidon Brevets & Stratégie
(86) Numéro de dépôt international: PCT/EP2011/060558
(87) Numéro de publication internationale: WO 2012/013429

(56) Documents cités:
- EP-A1- 1 911 347
- WO-A1-2008/043156

## Description

### 1. Domaine de l'invention

Le domaine de l'invention est celui de l'identification et/ou du prélèvement de tissu des animaux.

Plus précisément, l'invention concerne un nouvel ensemble pour le marquage et/ou le prélèvement de tissu d'un animal, permettant notamment de protéger les parties mâle ou femelle d'un dispositif de marquage et/ou de prélèvement avant leur utilisation, et de faciliter leur installation sur un outil de marquage et/ou de prélèvement correspondant.

### 2. Art antérieur

L'identification du bétail a été rendue obligatoire dans de nombreux pays pour assurer le suivi du cheptel et garantir l'origine des animaux destinés notamment à la consommation.

Ainsi, afin d'assurer sa traçabilité, un animal est classiquement identifié au moyen d'un dispositif de marquage visuel et/ou électronique, encore appelé marque. Une telle marque est formée d'une partie mâle et d'une partie femelle de marquage, conçues pour s'associer de manière irréversible.

Par exemple, dans ce contexte d'identification animale, la partie mâle comprend une tige s'étendant à partir d'un support destiné à prendre appui sur une face de l'oreille de l'animal. L'extrémité de la tige se termine par une pointe conique ou tronconique, également appelée tête, délimitant un épaulement externe permettant de maintenir la pointe dans une cavité de réception de la partie femelle. Une telle pointe permet de perforer l'oreille de l'animal avant d'associer la partie mâle et la partie femelle.

Il est également connu de prélever du tissu animal lors de la pose de la marque, ou bien indépendamment de la pose de la marque.

Par exemple, lorsque ce prélèvement est effectué lors de la pose de la marque, la pointe peut être tronquée et permettre le passage d'un élément de prélèvement, de type aiguille de biopsie. Selon une variante, un élément de prélèvement de type emporte-pièce peut être prévu à l'extrémité de la pointe tronquée.

Lorsque ce prélèvement est effectué indépendamment de la pose de la marque, un élément de prélèvement de type emporte-pièce peut être fixé à un outil de prélèvement comme une pince par exemple.

Dans toutes ces applications (marquage de l'animal et/ou prélèvement de tissus de l'animal), il est nécessaire de manipuler plusieurs éléments pour pouvoir poser la marque ou prélever un échantillon de tissu.

Ainsi, pour apposer une marque à un animal, il est nécessaire de monter la partie mâle de marquage sur une première branche d'un outil de marquage, de monter la partie femelle de marquage sur une deuxième branche de l'outil de marquage, éventuellement de tremper l'ensemble dans un produit désinfectant, avant de pouvoir réaliser l'opération de marquage.

Pour prélever un échantillon de tissu animal et apposer simultanément une marque, il est nécessaire de monter la partie mâle de marquage et de prélèvement (comprenant une tige et un élément de prélèvement) sur une première branche d'un outil de marquage et de prélèvement, de monter la partie femelle de marquage et de réception de tissu sur une deuxième branche de l'outil de marquage et de prélèvement, éventuellement de tremper l'ensemble dans un produit désinfectant, avant de pouvoir réaliser les opérations de marquage et de prélèvement.

Pour prélever un échantillon de tissu animal sans apposer simultanément une marque, il est nécessaire de monter la partie mâle de prélèvement (comprenant un élément de prélèvement) sur une première branche d'un outil de prélèvement, de monter la partie femelle de réception de tissu sur une deuxième branche de l'outil de prélèvement, éventuellement de tremper l'ensemble dans un produit désinfectant, avant de pouvoir réaliser l'opération de prélèvement.

Ces opérations de montage des différents éléments sur l'outil de marquage et/ou de prélèvement sont fastidieuses pour l'utilisateur.

Il existe donc un risque qu'un élément à monter sur l'outil tombe, et soit perdu ou contaminé par l'environnement extérieur (poussière, humidité, bactéries, etc).

De plus, il existe un risque accru que l'utilisateur se blesse lors de la manipulation de ses différents éléments, notamment lors de la manipulation de l'élément de prélèvement ou de la pointe de la partie mâle.

Il existe donc un besoin pour un nouveau conditionnement de ces éléments, qui simplifie leur montage.

Le document EP-A1-1911347 décrit un tel dispositif de marquage.

### 30 3. Exposé de l'invention

L'invention propose une solution nouvelle sous la forme d'un ensemble de marquage et/ou de prélèvement de tissu d'un animal, comprenant :
- une partie mâle de marquage et/ou de prélèvement de tissu,
- une partie femelle de marquage et/ou de réception dudit tissu.

Selon l'invention, un tel ensemble comprend un élément intermédiaire de liaison entre les parties mâle et femelle, présentant une première portion de solidarisation à ladite partie mâle et une deuxième portion de solidarisation à ladite partie femelle permettant de maintenir lesdites parties mâle et femelle selon un même axe de marquage et/ou de prélèvement, préalablement au marquage et/ou au prélèvement de tissu dudit animal, ledit élément intermédiaire de liaison étant amovible. En particulier, la première portion de solidarisation comprend au moins un logement recevant au moins partiellement la pointe et/ou l'extrémité d'un élément de prélèvement de la partie mâle.L'invention propose ainsi un ensemble « monobloc », selon lequel la partie mâle de marquage et/ou de prélèvement de tissu et la partie femelle de marquage et/ou de réception de tissu se trouvent maintenues coaxialement préalablement à l'opération de montage sur un outil de marquage et/ou de prélèvement.

En d'autres termes, la partie mâle, l'élément intermédiaire de liaison, et la partie femelle présentent un même axe, qui s'étend selon une seule direction (qui peut être légèrement courbe). L'élément intermédiaire de liaison offre donc un lien rigide assurant l'alignement entre les parties mâle et femelle.

L'utilisation d'un élément intermédiaire de liaison selon l'invention présente de nombreux avantages.

Tout d'abord, l'élément intermédiaire de liaison permet de maintenir ensemble la partie mâle et la partie femelle correspondante préalablement à l'identification et/ou au prélèvement de tissu animal. De cette façon, l'élément intermédiaire de liaison facilite le transport et la manipulation des parties mâle et femelle. De plus, on diminue les risques d'erreur pouvant survenir en utilisant une partie mâle et une partie femelle qui ne lui correspond pas (qui ne porte pas le même identifiant par exemple). Enfin, le pré-assemblage (en position de montage) des parties mâle et femelle permet de simplifier le montage des différents éléments sur l'outil de marquage et/ou de prélèvement, puisque seul l'ensemble monobloc est à manipuler.

Par ailleurs, l'élément intermédiaire de liaison permet de protéger les parties mâle et/ou femelle. En particulier, cet élément de liaison permet de protéger la pointe ou l'élément de prélèvement de l'environnement extérieur (poussière, humidité, bactéries, etc). Il permet notamment de protéger la pointe ou l'élément de prélèvement (en particulier son arête coupante) contre les altérations ou les souillures. On conserve ainsi une bonne qualité de perforation de la pointe ou de coupe de l'élément de prélèvement. De plus, l'utilisation d'un tel élément intermédiaire de liaison évite à l'utilisateur de se blesser lors de la manipulation de la pointe ou de l'élément de prélèvement.

Pour ce faire, selon un premier aspect, la première portion de solidarisation recouvre au moins une pointe et/ou l'extrémité d'un élément de prélèvement de la partie mâle.

Par exemple, l'orifice d'entrée du logement comprenant au moins un rebord permettant de maintenir la pointe et/ou l'élément de prélèvement dans l'élément intermédiaire de liaison.

Ainsi, la partie mâle peut être emboîtée dans l'élément intermédiaire de liaison pour solidariser la partie mâle de marquage et/ou de prélèvement et l'élément de liaison intermédiaire. Il est aussi possible de protéger la pointe et/ou l'élément de prélèvement lors des manipulations antérieures au marquage et/ou au prélèvement grâce à l'élément intermédiaire de liaison, puis de retirer (manuellement ou automatiquement) l'élément intermédiaire de liaison une fois que la partie mâle et la partie femelle sont montées sur l'outil de marquage et/ou de prélèvement.

Selon un deuxième aspect, la deuxième portion de solidarisation est insérée dans un tube de prélèvement et/ou une cavité de réception de la partie femelle.

Ainsi, l'élément intermédiaire de liaison peut être emboîté dans la partie femelle de marquage et/ou de réception pour solidariser la partie femelle et l'élément de liaison intermédiaire. Ceci permet aussi de protéger la partie femelle, en éviter que des corps étrangers viennent se loger dans la cavité de réception ou dans le tube de prélèvement de la partie femelle. Il n'est ainsi pas nécessaire de fermer un tel tube par un opercule préalablement au prélèvement.

Pour ce faire, selon un mode de réalisation particulier, la deuxième portion de solidarisation est cylindrique et présente un diamètre légèrement inférieur au diamètre du tube de prélèvement et/ou de la cavité de réception de la partie femelle.

Selon une variante, la deuxième portion de solidarisation recouvre l'extrémité ouverte d'un tube de prélèvement ou d'une tête de tube.

Selon cette variante, concernant plus spécifiquement les opérations de prélèvement, un tube ou une tête de tube peut être emboîté dans l'élément intermédiaire de liaison pour solidariser la partie femelle de prélèvement et l'élément de liaison intermédiaire.

Avantageusement, la partie mâle, l'élément intermédiaire de liaison et la partie femelle présentent une forme de révolution, autour d'un même axe de révolution.

Selon un autre aspect, l'élément intermédiaire de liaison est au moins partiellement déformable.

De cette façon, il est plus facile de le mettre en place lors de la fabrication de l'ensemble et de le retirer avant l'opération de marquage et/ou de prélèvement.

Par exemple, l'élément intermédiaire de liaison est réalisé en un matériau plastique légèrement déformable, comme le polypropylène (PP), le polystyrène (PS), le polyéthylène (PE), ou bien encore en un matériau plastique biodégradable, comme le polylactide (PLA) ou l'amidon (PCL).On note que la géométrie de l'élément intermédiaire de liaison peut être adaptée pour faciliter la déformation.

Selon un mode de réalisation particulier, l'élément intermédiaire de liaison est une pince comprenant au moins une partie mobile.

Les parties mâle et femelle sont alors maintenues ensemble par les parties mobiles de la pince.

En particulier, une telle pince comprend des moyens de préhension, formant un levier permettant d'actionner la ou les parties mobiles de la pince.

Il est ainsi facile de manipuler l'ensemble, de l'installer sur l'outil de marquage et/ou de prélèvement, et de désolidariser les partie mâle et femelle grâce à ces moyens de préhension.

Avantageusement, l'élément intermédiaire de liaison contient un agent spécifique destiné à être appliqué au moins partiellement sur la partie mâle et/ou la partie femelle, comme : un agent désinfectant, un agent cicatrisant, un agent conservateur, un agent dessiccant, un médicament, un vaccin, une combinaison d'au moins deux des agents précédents.

Ainsi, par exemple, la pointe ou l'élément de prélèvement de la partie mâle est en contact avec un agent désinfectant ou antiseptique lorsqu'elle est logée dans l'élément intermédiaire de liaison. En plus de protéger l'utilisateur de la pointe ou l'arête coupante de l'élément de prélèvement, cet aspect permet d'aseptiser la partie mâle avant utilisation, ce qui diminue le risque de contamination de la plaie de l'animal et permet une cicatrisation améliorée.

En particulier, un tel agent spécifique peut se présenter sous différentes formes, comme un gel, une crème, une graisse, un liquide, une poudre, un gaz, une mousse imprégnée, etc.

L'invention concerne par ailleurs un outil de marquage et/ou de prélèvement de tissu d'un animal destiné à coopérer avec un ensemble tel que décrit précédemment.

Selon l'invention, un tel outil comprend des moyens de désolidarisation de ladite partie mâle et de ladite partie femelle.

Ainsi, une fois l'ensemble monté sur une des branches par l'intermédiaire de la partie mâle ou de la partie femelle, l'outil selon l'invention permet de « récupérer » l'autre partie sur l'autre branche sans que l'utilisateur ait à manipuler l'ensemble.

Selon un mode de réalisation particulier, les moyens de désolidarisation comprennent des moyens de verrouillage de ladite partie femelle audit outil, permettant de solidariser ledit ensemble audit outil.

Par exemple, ces moyens de verrouillage sont de type bague de verrouillage, élément translatant, pédale, etc.

Selon ce mode de réalisation particulier, les moyens de désolidarisation comprennent également des moyens d'accrochage activés par une action sur ledit outil, permettant de solidariser ladite partie mâle audit outil et de désolidariser ladite partie mâle dudit élément intermédiaire de liaison ou de ladite partie femelle.

Par exemple, ces moyens d'accrochage sont de type moyens de serrage, de clippage, etc.

Ainsi, dans un premier temps, on monte l'ensemble sur une branche de l'outil, et dans un deuxième temps, on actionne l'outil ce qui permet de désolidariser les parties mâle et femelle.

Selon une autre caractéristique, l'outil comprend des moyens d'éjection de l'élément intermédiaire de liaison.

De cette façon, l'utilisateur n'a pas à manipuler l'élément intermédiaire de liaison, ce qui évite à nouveau le risque de blessure et de contamination de la pointe ou l'élément de prélèvement.

### 4. Liste des figures

D'autres caractéristiques et avantages de l'invention apparaîtront plus clairement à la lecture de la description suivante d'un mode de réalisation particulier, donné à titre de simple exemple illustratif et non limitatif, et des dessins annexés, parmi lesquels :
- les figures 1A à 1C illustrent un premier exemple d'ensemble selon l'invention, pouvant être utilisé pour marquer un animal ;
- les figures 2A à 2C illustrent un deuxième exemple d'ensemble selon l'invention, pouvant être utilisé pour marquer un animal et prélever simultanément un échantillon de tissu animal ;
- les figures 3A à 3C illustrent un troisième exemple d'ensemble selon l'invention, pouvant être utilisé pour prélever un échantillon de tissu animal, indépendamment de la pose d'une marque d'identification ;
- les figures 4A à 4C présentent différentes positions d'un outil de prélèvement, lors du montage des parties mâle et femelle de l'ensemble sur l'outil ;
- les figures 5A et 5B illustrent un autre exemple d'outil de marquage et de prélèvement.

### 5. Description d'un mode de réalisation de l'invention

### 5.1 Principe général

Le principe général de l'invention repose sur l'utilisation d'un élément intermédiaire de liaison permettant de solidariser une partie mâle de marquage et/ou de prélèvement de tissu et une partie femelle de marquage et/ou de réception de tissu, préalablement aux opérations de marquage et/ou de prélèvement.

De cette façon, la manipulation, le transport, etc, des parties mâle et femelle, et leur montage sur un outil de marquage et/ou de prélèvement, se trouvent facilités.

Plus précisément, un tel élément intermédiaire de liaison permet d'assurer une liaison rigide ou semi-rigide entre les partie mâle et femelle, et donc de maintenir ces parties mâle et femelle dans un même alignement.

On entend ici et par la suite par « partie mâle » :
- une partie mâle formée d'une tige s'étendant à partir d'un support et se terminant par une pointe si l'on se place dans un contexte de marquage animal (mise en place d'une marque visuelle et/ou électronique),
- un élément de prélèvement de type aiguille de biopsie ou emporte-pièce, si l'on se place dans un contexte de prélèvement de tissu animal (prélèvement mis en oeuvre indépendamment de la mise en place d'une marque visuelle et/ou électronique),
- une partie mâle comprenant une tige s'étendant à partir d'un support et comprenant un élément de prélèvement de type aiguille de biopsie ou emporte-pièce, si l'on se place dans un contexte de marquage et de prélèvement de tissu animal (prélèvement mis en oeuvre conjointement à la mise en place d'une marque visuelle et/ou électronique) ;
   et par « partie femelle » :
- une partie femelle comprenant une cavité de réception si l'on se place dans un contexte de marquage animal,
- un tube de prélèvement si l'on se place dans un contexte de prélèvement de tissu animal,
- une partie femelle comprenant une cavité de réception et/ou un tube de prélèvement si l'on se place dans un contexte de marquage et de prélèvement de tissu animal.

### 5.2 Application au marquage animal

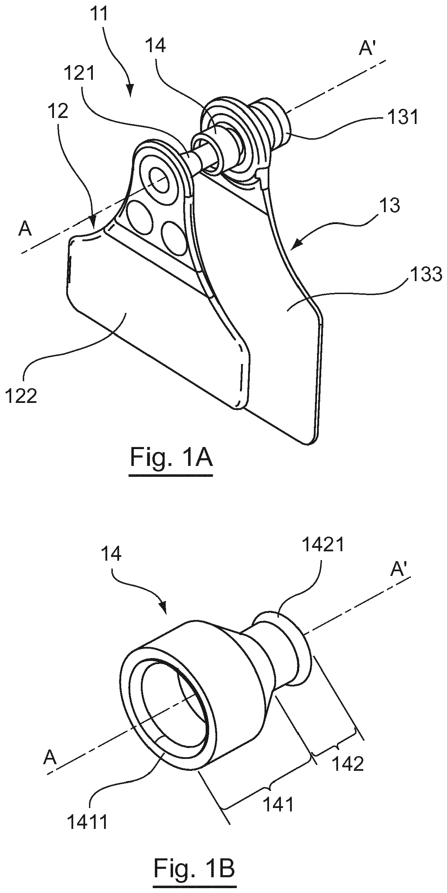

On décrit ci-après, en relation avec les figures 1A à 1C, un premier exemple d'ensemble 11 selon l'invention, pouvant être utilisé dans un contexte de marquage animal (mise en place d'une marque visuelle et/ou électronique).

Comme illustré en figure 1A, un tel ensemble 11 comprend une partie mâle 12, une partie femelle 13, et un élément intermédiaire de liaison 14.

Les parties mâles 12 et femelle 13 sont classiques.

Par exemple, la partie mâle 12 comprend une tige 121 s'étendant à partir d'un support de marquage 122. La tige 121 est terminée par une pointe conique ou tronconique 123 (également appelée tête) délimitant un épaulement 124 externe au niveau de sa base. La pointe 123 permet de perforer la peau de l'animal avant d'associer la partie mâle 12 avec la partie femelle 13.

La partie femelle 13 comprend quant à elle un bouton de réception 131 présentant une cavité 132 pour l'introduction de la pointe 123 de la tige 121. Des lamelles 134 sont prévues au niveau de l'orifice d'entrée de la cavité 132, permettant le passage de la pointe 123 dans un sens pour venir insérer la pointe 123 de la partie mâle dans la cavité 132, et empêchant son extraction dans l'autre sens.

La base de la partie femelle forme également un support de marquage 133.

Selon l'invention, la partie mâle 12 et la partie femelle 13 sont maintenues coaxialement selon l'axe de marquage AA' grâce à l'élément intermédiaire de liaison 14. En d'autres termes, l'élément intermédiaire de liaison permet de solidariser les parties mâle 12 et femelle 13 de sorte que l'axe de révolution de la tige 121 et l'axe de révolution de la cavité de réception 132 soient confondus.

Plus précisément, comme illustré en figure 1B, l'élément intermédiaire de liaison 14 présente une première portion 141 de solidarisation à la partie mâle 12 et une deuxième portion de solidarisation 142 à la partie femelle 13, permettant de maintenir ensemble les parties mâle et femelle, préalablement au marquage de l'animal.

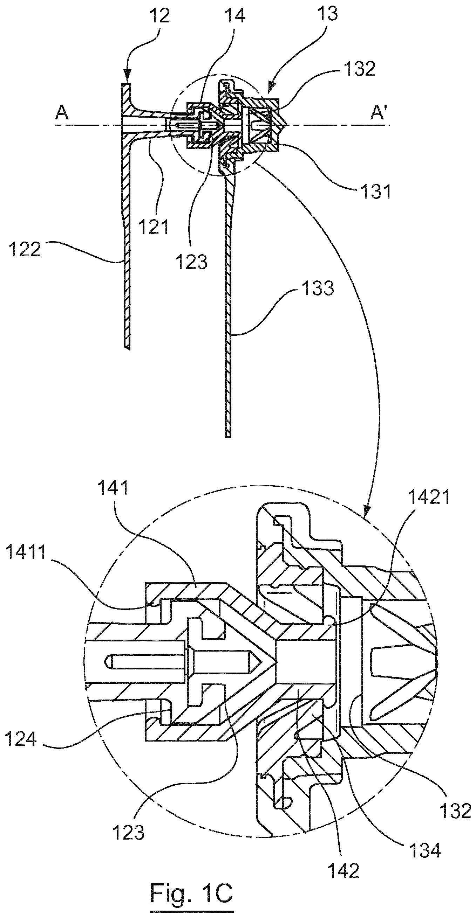

Selon cet exemple, et comme illustré en figure 1C, la première portion de solidarisation 141 recouvre la pointe 123 de la partie mâle 12. Plus précisément, la première portion de solidarisation 141 définit un logement présentant une forme adaptée pour recevoir la pointe 123. Le diamètre interne du logement est donc légèrement supérieur à celui de la base de la pointe 123 (correspondant à la partie la plus large de la pointe), afin que celle-ci puisse pénétrer dans le logement. De plus, l'orifice d'entrée du logement comprend au moins un rebord intérieur 1411 permettant de maintenir la pointe 123 dans l'élément intermédiaire de liaison 14, l'épaulement 124 de la pointe 123 venant en butée contre le rebord intérieur 1411 lorsque la pointe 123 est insérée dans l'élément intermédiaire de liaison. De cette façon, l'élément de liaison permet de protéger la pointe de l'environnement extérieur (poussière, humidité, bactéries, etc).

La deuxième portion de solidarisation 142 est quant à elle insérée dans la cavité de réception 132 de la partie femelle 13. Plus précisément, la deuxième portion de solidarisation 142 présente une forme adaptée pour coopérer avec la cavité de réception 132 la pointe 123. Elle est par exemple cylindrique et présente un diamètre légèrement inférieur au diamètre de la cavité de réception 132 de la partie femelle. Elle comprend également au moins un rebord extérieur 1421, permettant de maintenir l'élément intermédiaire de liaison 14 dans la cavité de la partie femelle, les lamelles 134 de la partie femelle prenant appui sur le rebord extérieur 1421 pour retenir l'élément intermédiaire de liaison 14 dans la partie femelle. Avantageusement, le diamètre de la base de la pointe 123 est supérieur au diamètre du rebord extérieur 1421 de la deuxième portion de solidarisation 1421, de façon à ne pas détériorer les lamelles 134 lors du retrait de l'élément intermédiaire de solidarisation 14 de la partie femelle.

L'élément intermédiaire de liaison 14 ou les rebords intérieur 1411 et extérieur 1421 peuvent être réalisés dans un matériau légèrement déformable, tel que le polypropylène, le polystyrène, le polyéthylène, afin de faciliter l'insertion et le retrait de la partie mâle 12 dans la première section de solidarisation 141 et l'insertion et le retrait de la deuxième section de solidarisation 142 dans la partie femelle 13. L'élément intermédiaire de liaison 14 doit également être suffisamment rigide pour maintenir la partie mâle et la partie femelle co-axialement, notamment lors du transport de l'ensemble et de sa mise en place sur un outil de marquage.

On rappelle que l'élément intermédiaire de liaison 14 est amovible. Il peut donc être retiré juste avant l'opération de marquage de l'animal.Il est alors nécessaire d'exercer une légère force pour pouvoir libérer les parties mâle 12 et femelle 13 de l'élément de liaison 14.

De plus, avantageusement, un agent spécifique est prévu à l'intérieur de l'élément intermédiaire de liaison 14. Il peut s'agir d'un agent désinfectant, d'un agent cicatrisant, d'un médicament, d'un vaccin, etc. Un tel agent permet ainsi de détruire au moins en partie les micro-organismes (bactéries, virus, etc) pouvant provoquer des infections au niveau des tissus perforés lors de la pose de la marque, ou de soigner ou traiter l'animal en cas de besoin, cet agent pouvant passer dans le sang au niveau des tissus perforés ou être absorbé par la peau de l'animal.

En particulier, un tel agent spécifique peut se présenter sous différentes formes, comme un gel, une crème, une graisse, un liquide, une poudre, un gaz, une mousse imprégnée, etc, éventuellement contenu dans un réservoir ou fermé par un opercule percé par la pointe de la partie mâle.

### 5.3 Application au prélèvement combiné au marquage

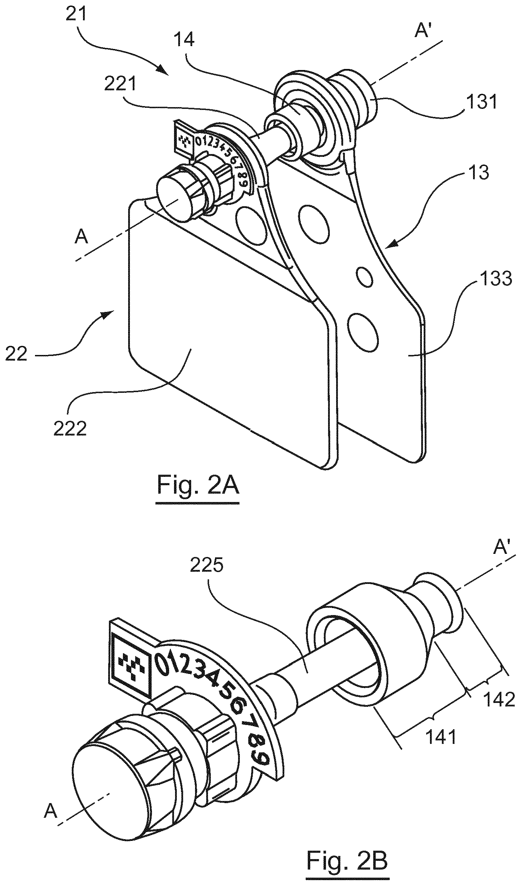

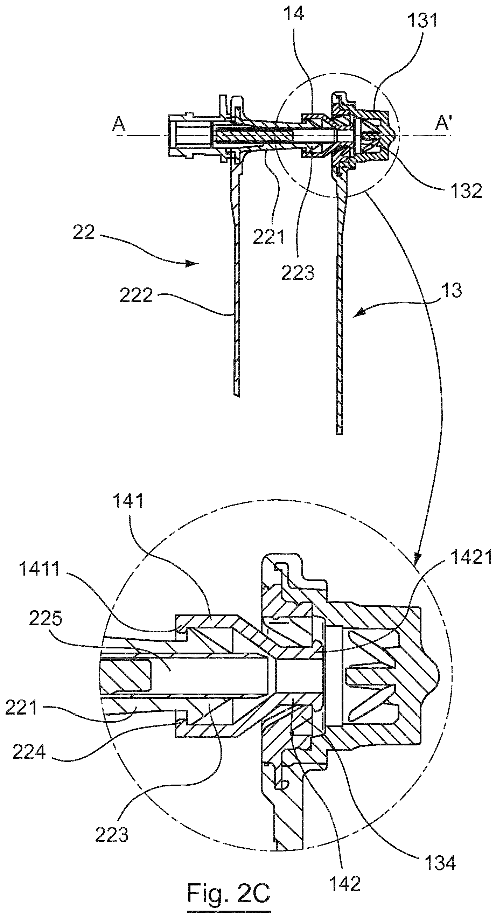

On décrit ci-après, en relation avec les figures 2A à 2C, un deuxième exemple d'ensemble 21 selon l'invention, pouvant être utilisé dans un contexte de marquage et de prélèvement de tissu animal.

Comme illustré en figure 2A, un tel ensemble 21 comprend une partie mâle 22, une partie femelle 13, et un élément intermédiaire de liaison 14.

Selon cet exemple, la partie mâle 22 comprend une tige 221 s'étendant à partir d'un support de marquage 222. La tige 221 est terminée par une pointe conique ou tronconique 223 tronquée, délimitant un épaulement externe 224 au niveau de sa base. La tige 223 permet le passage d'un élément de prélèvement de tissu animal, comme une aiguille de biopsie 225 ou un emporte-pièce.

La partie femelle 13 peut être similaire à la partie femelle décrite en relation avec les figures 1A et 1B.

Selon l'invention, la partie mâle 22 et la partie femelle 13 sont maintenues coaxialement selon l'axe de marquage et de prélèvement AA' grâce à l'élément intermédiaire de liaison 14. En d'autres termes, l'élément intermédiaire de liaison permet de solidariser les parties mâle 22 et femelle 13 de sorte que l'axe de révolution de la tige 221 et l'axe de révolution de la cavité de réception 132 soient confondus.

L'élément intermédiaire de liaison 14 est similaire à celui décrit en relation avec les figures 1A à 1C. Plus précisément, comme illustré en figures 1B et 1C, la première portion de solidarisation 141 recouvre la pointe tronquée 223 de la partie mâle 22 et l'extrémité de l'élément de prélèvement 225. La première portion de solidarisation 141 définit ici un logement présentant une forme adaptée pour recevoir la pointe 223 et l'extrémité de l'élément de prélèvement 225.

Ainsi, l'élément intermédiaire de liaison 14 permet de protéger la pointe et l'élément de prélèvement contre les altérations ou les souillures. On conserve ainsi selon l'invention une bonne qualité de coupe de l'arête coupante de l'élément de prélèvement.

Comme indiqué précédemment, un agent spécifique peut être prévu à l'intérieur de l'élément intermédiaire de liaison 14. Ainsi, comme illustré en figure 2B, l'arête coupante de l'élément de prélèvement peut être en contact avec un agent désinfectant, cicatrisant, etc, avant l'opération de prélèvement.

Dans un contexte de marquage et de prélèvement, un tel agent peut également être un agent conservateur ou dessicant, permettant d'améliorer la conservation d'un échantillon de tissu à prélever, et éventuellement de le préparer en vue d'un traitement ultérieur comme une analyse ADN.

En d'autres termes, l'agent spécifique selon l'invention peut prendre la forme de tout produit pouvant agir sur l'animal ou sur un échantillon de tissu prélevé de l'animal.

### 5.4 Application au prélèvement

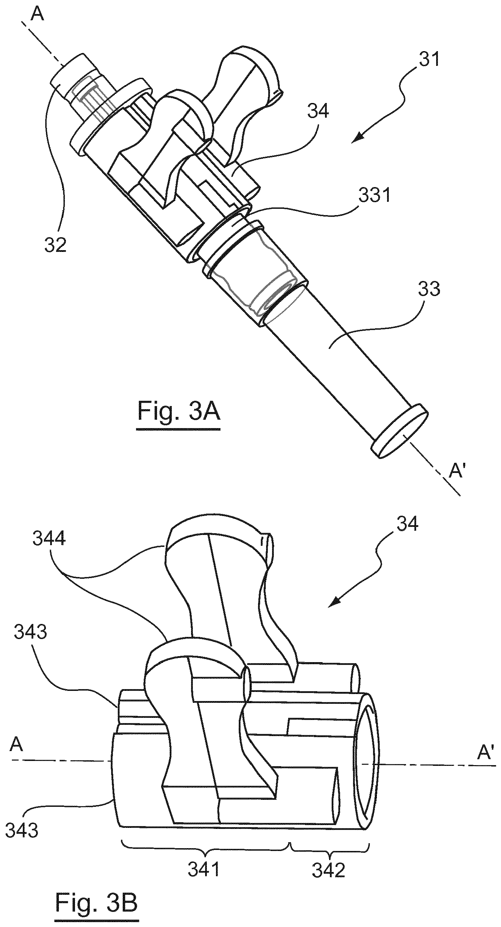

On décrit ci-après, en relation avec les figures 3A à 3C, un troisième exemple d'ensemble 31 selon l'invention, pouvant être utilisé dans un contexte de prélèvement de tissu animal (mise en oeuvre indépendamment de la pose d'une marque visuelle et/ou électronique).

Comme illustré en figure 3A, un tel ensemble 31 comprend une partie mâle 32 comprenant un élément de prélèvement, une partie femelle 33 comprenant un tube de prélèvement, et un élément intermédiaire de liaison 34.

Par exemple, l'élément de prélèvement comprend un élément de coupe 321 destiné à découper un échantillon de tissu de l'animal et un élément poussoir 322 mobile par rapport à l'élément de coupe 321, permettant de pousser l'échantillon dans un tube de prélèvement 33 après découpe de l'échantillon par l'élément de coupe 321.

Le tube de prélèvement 33 est quant à lui équipé d'une tête de tube 331. En particulier, une telle tête de tube procure un support sur lequel l'élément de coupe 321 peut s'appuyer pour découper correctement les tissus de l'animal. Elle permet également la fermeture du tube, par exemple par emboîtement ou clippage de l'élément poussoir 322 et/ou de l'élément de coupe 321 dans la tête de tube 331, ainsi que l'automatisation de l'ouverture des tubes par les laboratoires d'analyse, en décapsulant la tête de tube de façon que l'échantillon reste à l'intérieur du tube.

Un élément de prélèvement et un tube de prélèvement selon ce troisième exemple sont notamment décrits dans la demande de brevet français FR 2 939 281 au nom du même Demandeur.

Selon l'invention, la partie mâle (élément de prélèvement) 32 et la partie femelle (tube de prélèvement) 33 sont maintenues co-axialement, selon l'axe de prélèvement AA', grâce à l'élément intermédiaire de liaison 34. En d'autres termes, l'élément intermédiaire de liaison permet de solidariser les parties mâle 32 et femelle 33 de sorte que l'axe de révolution de l'élément de prélèvement 32 et l'axe de révolution du tube de prélèvement 33 soient confondus.

Plus précisément, comme illustré en figure 3B, l'élément intermédiaire de liaison 34 présente une première portion 341 de solidarisation à la partie mâle 32 et une deuxième portion de solidarisation 342 à la partie femelle 33, permettant de maintenir ensemble les parties mâle et femelle, préalablement au prélèvement de tissu animal.

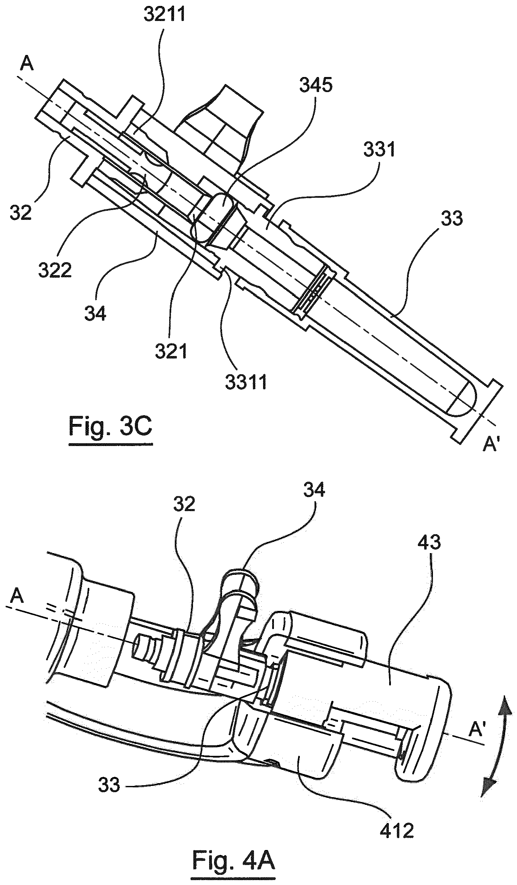

Selon cet exemple, et comme illustré en figure 3C, la première portion de solidarisation 341 recouvre l'élément de coupe 321 de la partie mâle 32. Plus précisément, la première portion de solidarisation 341 définit un logement présentant une forme adaptée pour recevoir l'élément de coupe 321, et éventuellement une partie de son support 3211.

La deuxième portion de solidarisation 342 recouvre quant à elle l'extrémité ouverte du tube de prélèvement 33 ou de la tête de tube 331. Selon l'exemple illustré, la deuxième portion de solidarisation 342 définit un logement présentant une forme adaptée pour recevoir la tête de tube 331. Si la tête de tube est munie d'une collerette 3311, la deuxième portion de solidarisation peut prévoir un renfoncement destiné à recevoir cette collerette.

En particulier, selon ce troisième exemple, l'élément intermédiaire de liaison 34 est une pince, comprenant deux parties mobiles 343 et des moyens de préhension 344. Plus précisément, les moyens de préhension forment un levier permettant d'actionner les parties mobiles de la pince. Ainsi, une action d'un utilisateur sur les moyens de préhension 344 permet d'écarter les deux parties mobiles 343 de la pince. Il est alors possible d'introduire la partie mâle 32 et la partie femelle 33 entre les parties mobiles 343. Lorsque l'utilisateur relâche son action, les parties mobiles se resserrent, permettant ainsi de maintenir ensemble la partie mâle 32 et la partie femelle 33, selon l'axe de prélèvement AA'. Par exemple, chaque partie mobile prend la forme d'un demi-cylindre creux, la fermeture de la pince définissant un cylindre encapsulant au moins partiellement l'élément de coupe 321 et la tête de tube 331.

Selon ce troisième exemple, l'élément intermédiaire de liaison 34 peut être réalisé dans un matériau plastique tel que le polypropylène, le polystyrène, le polyéthylène ou bien encore dans un matériau plastique biodégradable.

Avantageusement, un agent spécifique est prévu à l'intérieur de l'élément intermédiaire de liaison 34. Il peut s'agir d'un agent désinfectant, d'un agent cicatrisant, d'un agent conservateur, d'un agent dessiccant, d'un médicament, d'un vaccin, d'une combinaison d'au moins deux des agents précédents, etc.

Par exemple, l'agent spécifique est un désinfectant, imprégnant une mousse 345. Lors de la fermeture de la pince 34 sur les parties mâle 32 et femelle 33, la mousse est légèrement comprimée et libère une substance désinfectante, permettant de désinfecter l'arête coupante de l'élément de coupe 321 et l'orifice d'entrée de la tête de tube 331.

### 5.5 Mise en place de l'ensemble sur un outil de marquage et/ou de prélèvement

Comme déjà indiqué, en plus d'améliorer la sécurité et la qualité de perçage de la pointe ou de coupe de l'élément de prélèvement, l'utilisation d'un ensemble selon l'invention permet de simplifier la livraison et la manipulation des parties mâle et femelle, ainsi que leur mise en place sur un outil de marquage et/ou de prélèvement.

On présente ci-après, en relation avec les figures 4A à 4C, les différentes étapes mises en oeuvre pour la mise en place des parties mâle et femelle sur un outil de prélèvement. Bien entendu, des opérations similaires peuvent être mises en oeuvre pour une mise en place sur un outil de marquage.

Par exemple, un tel outil comprend une partie fixe encore appelée corps 41, définissant notamment une première poignée, et une partie articulée encore appelée levier 42, définissant une deuxième poignée.

Le corps 41 de l'outil définit également deux branches, dont une première branche 411 est destinée à coopérer avec la partie mâle 32 et une deuxième branche 412 destinée à coopérer avec une partie femelle 33, telles qu'illustrées en figure 3A par exemple.

Plus précisément, comme illustré en figure 4A, l'utilisateur monte l'ensemble 31 « monobloc » selon l'invention sur la deuxième branche 412 au cours d'une première étape. On rappelle que cet ensemble 31 comprend une partie mâle 32, formée d'un élément de prélèvement, un élément intermédiaire de liaison 34 et une partie femelle 33, formée d'un tube de prélèvement comprenant une tête de tube 331, maintenus ensemble selon l'axe de prélèvement AA'.

Pour ce faire, la deuxième branche 412 comprend des moyens de verrouillage du tube de prélèvement 33 permettant de solidariser l'ensemble 31 à l'outil, comme une bague de verrouillage 43 par exemple.

La partie mâle 32, l'élément intermédiaire de liaison 34 et la partie femelle 33 se trouvent alors solidarisés à la deuxième branche 412 de l'outil.

Afin de désolidariser les parties mâle 32 et femelle 33, une première action peut être exercée sur l'outil de prélèvement, par exemple sur une course réduite du levier 42. En d'autres termes, l'utilisateur peut effectuer une légère pression sur le levier 42, pour désolidariser les parties mâle 32 et femelle 33 et « armer » l'outil de prélèvement. On note que l'outil peut être actionné manuellement ou au moyen d'une énergie électrique, pneumatique, ou autre.

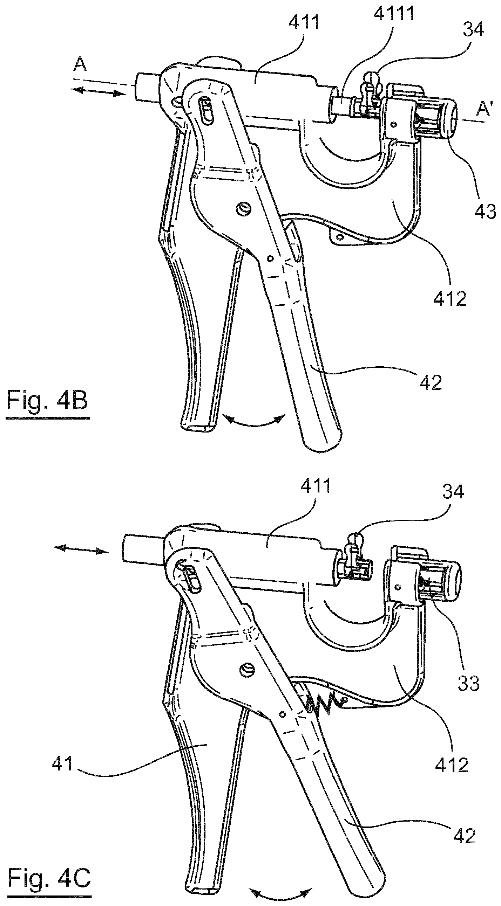

Comme illustré en figure 4B, une action sur le levier 42 entraîne des premiers moyens d'entraînement 4111. Par exemple, ces premiers moyens d'entraînement prennent la forme d'un pointeau, guidé en translation ou en rotation selon l'axe de prélèvement AA'.

Un tel pointeau 4111 comprend des moyens d'accrochage, permettant de solidariser la partie mâle 32 au pointeau. Le pointeau récupère ainsi la partie mâle 32, par exemple par effet de serrage ou de clippage.

Par exemple, le pointeau comprend une section de diamètre légèrement inférieur au diamètre intérieur de l'élément de prélèvement ou d'un support de l'élément de prélèvement. Cette section de diamètre inférieur peut ainsi venir s'insérer à force dans l'élément de prélèvement, au niveau de sa base (extrémité opposée à l'arête tranchante), pour solidariser la partie mâle à l'outil.

Selon un autre exemple, le pointeau comprend une portion qui peut se dilater pour venir enserrer la base de la partie mâle.Comme illustré en figure 4C, lorsque l'utilisateur relâche la pression sur le levier 42 par exemple, le pointeau 4111 revient dans sa position initiale, entraînant avec lui la partie mâle 32 et éventuellement l'élément intermédiaire de liaison 34. En plus de solidariser la partie mâle 32 au pointeau, les moyens d'accrochage permettent ainsi de désolidariser la partie mâle 32 de l'élément intermédiaire de liaison 34 ou de la partie femelle 33.

Les parties mâle 32 et femelle 33 sont alors toutes les deux montées sur l'outil de prélèvement, sans que l'utilisateur ait eu à manipuler l'élément de prélèvement 33.

L'utilisateur peut alors retirer l'élément intermédiaire de liaison 34, qui permettait de protéger l'arête coupante de l'élément de prélèvement (protection en terme de sécurité pour l'utilisateur et protection en terme de qualité pour l'arête coupante), en appuyant sur les moyens de préhension 344 formant levier par exemple, ou en actionnant des moyens d'éjection de l'élément intermédiaire de liaison prévus sur l'outil.

Par exemple, de tels moyens d'éjection prennent la forme d'un levier indépendant en forme de fourchette, permettant de pousser ou tirer l'élément intermédiaire de liaison et l'extraire de la partie mâle ou femelle sur laquelle il est encore positionné.

L'élément intermédiaire de liaison 34 peut alors être jeté, ou bien conservé afin notamment d'être repositionné sur l'élément de prélèvement après l'opération de prélèvement. Un tel élément de liaison peut être conçu dans un matériau biodégradable, comme le polylactide (PLA) ou l'amidon (PCL).

Les parties mâle 32 et femelle 33 sont alors montées respectivement sur chacune des branches de l'outil de prélèvement, et l'outil de prélèvement est prêt pour l'opération de prélèvement.

Une telle phase préalable d'armement peut notamment être mise en oeuvre par un outil de prélèvement tel que décrit dans la demande de brevet français FR2961087 au nom du même Demandeur.

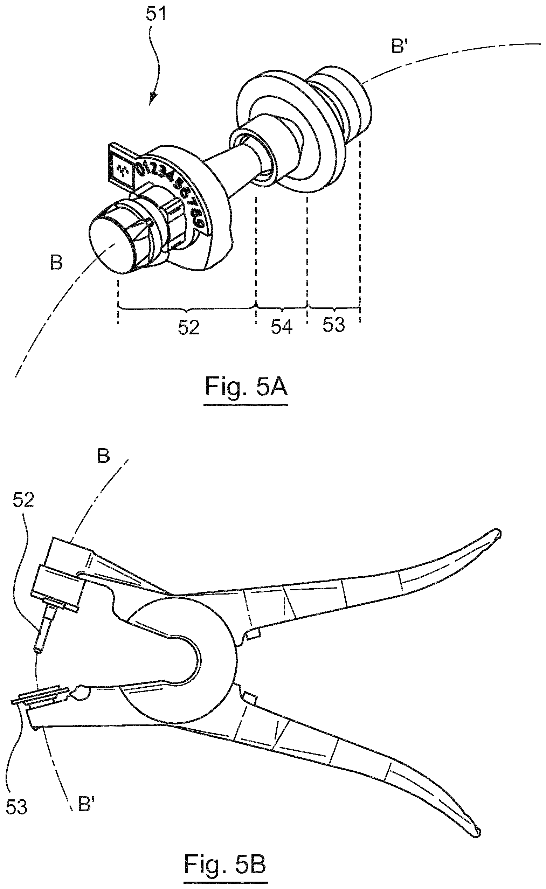

Bien entendu, des opérations similaires peuvent être mises en oeuvre pour une mise en place sur un autre outil, comme l'outil de marquage et de prélèvement illustré en figure 5B par exemple.

Dans ce cas, comme illustré en figure 5A, l'ensemble 51 comprend une partie mâle 52, un élément intermédiaire de liaison 54 et une partie femelle 53, maintenus ensemble selon un axe BB' légèrement incurvé, pour respecter l'axe de marquage et de prélèvement défini par la rotation des branches de l'outil.

## Revendications

1. Ensemble de marquage et/ou de prélèvement de tissu d'un animal,
comprenant :
- une partie mâle (12 ; 22 ; 32) de marquage et/ou de prélèvement de tissu,
- une partie femelle (13 ; 33) de marquage et/ou de réception dudit tissu, ledit ensemble comprenant un élément intermédiaire de liaison (14 ; 34) entre lesdites parties mâle et femelle,
ledit élément intermédiaire de liaison (14 ; 34) présentant une première portion de solidarisation (141 ; 341) à ladite partie mâle et une deuxième portion de solidarisation (142 ; 342) à ladite partie femelle permettant de maintenir lesdites parties mâle et femelle selon un même axe de marquage et/ou de prélèvement, préalablement au marquage et/ou au prélèvement de tissu dudit animal,
et ledit élément intermédiaire de liaison étant amovible, **caractérisé en ce que** ladite première portion de solidarisation (141 ; 341) comprend au moins un logement recevant au moins partiellement la pointe et/ou l'extrémité d'un élément de prélèvement de ladite partie mâle.

2. Ensemble selon la revendication 1, **caractérisé en ce que** l'orifice d'entrée dudit logement comprend au moins un rebord permettant de maintenir ladite pointe et/ou ledit élément de prélèvement dans ledit élément intermédiaire de liaison.

3. Ensemble selon l'une quelconque des revendications 1 et 2, **caractérisé en ce que** ladite deuxième portion de solidarisation (142) est insérée dans un tube de prélèvement et/ou une cavité de réception de ladite partie femelle.

4. Ensemble selon l'une quelconque des revendications 1 et 2, **caractérisé en ce que** ladite deuxième portion de solidarisation (342) recouvre l'extrémité ouverte d'un tube de prélèvement ou d'une tête de tube.

5. Ensemble selon l'une quelconque des revendications 1 à 4, **caractérisé en ce que** ledit élément intermédiaire de liaison (14 ; 34) est une pince comprenant au moins une partie mobile.

6. Ensemble selon la revendication 5, **caractérisé en ce que** ladite pince comprend des moyens de préhension, forment un levier permettant d'actionner la ou les parties mobiles de ladite pince.

7. Ensemble selon l'une quelconque des revendications 1 à 6, **caractérisé en ce que** ledit élément intermédiaire de liaison (14 ; 34) contient un agent spécifique destiné à être appliqué au moins partiellement sur ladite partie mâle et/ou ladite partie femelle.

8. Ensemble selon la revendication 7, **caractérisé en ce que** ledit agent spécifique appartient au groupe comprenant :
- un agent désinfectant ;
- un agent cicatrisant ;
- un agent conservateur ;
- un agent dessiccant ;
- un médicament ;
- un vaccin ;
- une combinaison d'au moins deux des agents précédents.

9. Outil de marquage et/ou de prélèvement de tissu d'un animal destiné à coopérer avec un ensemble selon l'une quelconque des revendications 1 à 8, **caractérisé en ce qu'**il comprend des moyens de désolidarisation de ladite partie mâle et de ladite partie femelle.

10. Outil selon la revendication 9, **caractérisé en ce que** lesdits moyens de désolidarisation comprennent des moyens de verrouillage de ladite partie femelle audit outil, permettant de solidariser ledit ensemble audit outil.

11. Outil selon l'une quelconque des revendications 9 et 10, **caractérisé en ce que** lesdits moyens de désolidarisation comprennent des moyens d'accrochage activés par une action sur ledit outil, permettant de solidariser ladite partie mâle audit outil et de désolidariser ladite partie mâle dudit élément intermédiaire de liaison ou de ladite partie femelle.

12. Outil selon l'une quelconque des revendications 9 à 11, **caractérisé en ce qu'**il comprend des moyens d'éjection dudit élément intermédiaire de liaison.

## Patentansprüche

1. Einheit der Markierung und/oder der Probennahme von Gewebe eines Tieres, umfassend:
- einen Einsteckteil (12; 22; 32) des Markierens und/oder der Probennahme von Gewebe,
- einen Aufnahmeteil (13; 33) des Markierens und/oder der Aufnahme des Gewebes,
wobei die Einheit ein Zwischenverbindungselement (14; 34) zwischen dem Einsteckteil und dem Aufnahmeteil aufweist,
wobei das Zwischenverbindungselement (14; 34) einen ersten Abschnitt der Verbindung (141; 341) mit dem Einsteckteil und einen zweiten Abschnitt der Verbindung (142; 342) mit dem Aufnahmeteil aufweist, die ermöglichen, vor dem Markieren und/oder der Probennahme des Gewebes des Tieres, den Einsteckteil und den Aufnahmeteil gemäß einer gleichen Achse zum Markieren und/oder zum Probennahme zu wahren,
wobei das Zwischenverbindungselement herausnehmbar ist,
**dadurch gekennzeichnet, dass**
der erste Abschnitt der Verbindung (141; 341) wenigstens eine Aufnahme aufweist, die die Spitze und/oder das Endstück eines Elements zur Probennahme des Einsteckteils aufnimmt.

2. Einheit nach Anspruch 1,
**dadurch gekennzeichnet, dass** die Erfindung des Eingangs der Aufnahme wenigstens einen Rand aufweist, der ermöglicht, die Spitze und/oder das Element zur Probennahme in dem Zwischenverbindungselement zu halten.

3. Einheit nach einem der Ansprüche 1 und 2,
**dadurch gekennzeichnet, dass** der zweite Abschnitt der Verbindung (142) in ein Probennahmerohr und/oder einen Aufnahmehohlraum des Aufnahmeteils eingefügt ist.

4. Einheit nach einem der Ansprüche 1 und 2,
**dadurch gekennzeichnet, dass** der zweite Abschnitt der Verbindung (342) den offenen Endteil eines Probennahmerohrs oder einen Kopf des Rohres umhüllt.

5. Einheit nach einem der Ansprüche 1 bis 4,
**dadurch gekennzeichnet, dass** das Zwischenverbindungselement (14, 34) eine Klammer ist, die wenigstens ein bewegbares Teil aufweist.

6. Einheit nach Anspruch 5,
**dadurch gekennzeichnet, dass** die Klammer Greifmittel aufweist, die einen Hebel bilden, der das Betätigen des oder der bewegbaren Teile der Klammer ermöglicht.

7. Einheit nach einem der Ansprüche 1 bis 6,
**dadurch gekennzeichnet, dass** das Zwischenverbindungselement (14; 34) einen spezifischen Wirkstoff enthält, der dazu vorgesehen ist, wenigstens teilweise auf den Einsteckteil und/oder den Aufnahmeteil angewandt zu werden.

8. Einheit nach Anspruch 7,
**dadurch gekennzeichnet, dass** das spezifische Mittel zu der Gruppe gehört umfassend:
- ein desinfizierendes Mittel;
- ein narbenbildendes Mittel;
- ein konservierendes Mittel;
- ein trocknendes Mittel;
- ein Medikament;
- einen Impfstoff;
- eine Kombination wenigstens zweier der vorstehenden Mittel.

9. Werkzeug des Markierens und/oder der Probennahme von Gewebe eines Tieres, das dazu vorgesehen ist, mit einer Einheit nach einem der Ansprüche 1 bis 8 zusammenzuwirken,
**dadurch gekennzeichnet, dass** es Mittel zum Trennen des Einsteckteils von dem Aufnahmeteil aufweist.

10. Werkzeug nach Anspruch 9,
**dadurch gekennzeichnet, dass** das Mittel zum Trennen Mittel zum Verriegeln des Aufnahmeteils an dem Werkzeug aufweisen, die ermöglichen, die Einheit an dem Werkzeug zu verbinden.

11. Werkzeug nach einem der Ansprüche 9 und 10,
**dadurch gekennzeichnet, dass** die Mittel zum Trennen Befestigungsmittel aufweisen, die durch eine Aktion auf dem Werkzeug aktiviert werden, die die Verbindung des Einsteckteils an dem Werkzeug und die Trennung des Einsteckteils von dem Zwischenverbindungselement oder dem Aufnahmeteil ermöglichen.

12. Werkzeug nach einem der Ansprüche 9 bis 11,
**dadurch gekennzeichnet, dass** es Mittel zum Auswerfen des Zwischenverbindungselementes aufweist.

## Claims

1. Unit for tagging and/or sampling animal tissue comprising:
- a male part (12; 22; 32) for tagging and/or sampling tissue,
- a female part (13; 33; 32) for tagging and/or receiving said tissue,
said unit comprising an intermediate linking element (14; 34) between said male and female parts,
said intermediate linking element (14; 34) having a first portion for joining (141; 341) to said male part and a second portion for joining (142; 342) to said female part enabling said male and female parts to be held in a same tagging and/sampling axis prior to the tagging and/or sampling of said animal tissue,
and said intermediate linking element being removable, **characterised in that** said first joining portion (141; 341) comprises at least one housing at least partially receiving the tip and/or the end of a sampling element of said male part.

2. Unit according to claim 1, **characterised in that** the input hole of said housing comprises at least one ledge enabling said tip and/or said sampling element to be held in said intermediate linking element.

3. Unit according to any one of claims 1 and 2, **characterised in that** said second joining portion (142) is inserted into a sampling tube and/or a receiving cavity of said female part.

4. Unit according to any one of claims 1 and 2, **characterised in that** said second joining portion (342) covers the open end of a sampling tube or a tube head.

5. Unit according to any one of claims 1 to 4, **characterised in that** said intermediate linking element (14; 34) is a clamp comprising at least one mobile part.

6. Unit according to claim 5, **characterised in that** said clamp comprises grasping means, forming a lever enabling the moving part or parts of said clamp to be actuated.

7. Unit according to any one of claims 1 to 6, **characterised in that** said intermediate linking element (14; 34) contains a specific agent intended to be applied at least partially to said male part and/or said female part.

8. Unit according to claim 7, **characterised in that** said specific agent belongs to the group consisting of:
- a disinfectant;
- a cicatrizing agent;
- a preserving agent;
- a desiccant;
- a medicine;
- a vaccine;
- a combination of at least two of the above agents.

9. Tool for tagging and/or sampling tissue from an animal designed to cooperate with a unit according to any one of claims 1 to 8, **characterised in that** it comprises means for disjoining said male part and said female part.

10. Tool according to claim 9, **characterised in that** said disjoining means comprise means for locking of said female part to said tool, enabling said unit to be rigidly connected to said tool.

11. Tool according to any one of claims 9 and 10, **characterised in that** said disjoining means comprise hooking means activated by action on said tool, enabling said male part to be rigidly connected to said tool and enabling said male part to be disconnected from said intermediate linking element or from said female part.

12. Tool according to any one of claims 9 to 11, **characterised in that** it comprises means for ejecting said intermediate linking element.
